# EUROPÄISCHE PATENTSCHRIFT

(11) **EP 0 731 799 B1**
(45) Veröffentlichungstag und Bekanntmachung des Hinweises auf die Patenterteilung: **15.07.1998**
(21) Anmeldenummer: 95902085.0
(22) Anmeldetag: 24.11.1994
(51) Int. Cl.: C07D 307/92

(54) **VERFAHREN ZUR HERSTELLUNG VON 8$g(a),12-OXIDO-13,14,15,16-TETRANORLABDAN**
PROCESS FOR THE PRODUCTION OF 8$g(a),12-OXIDO-13,14,15,16-TETRANORLABDANE
PROCEDE DE PRODUCTION DE 8$g(a)-12-OXYDO-13,14,15,16-TETRANORLABDANE

(30) Priorität: 03.12.1993 DE 4341275
(43) Veröffentlichungstag der Anmeldung: 18.09.1996
(73) Patentinhaber: Henkel Kommanditgesellschaft auf Aktien, 40191 Düsseldorf (DE)
(72) Erfinder: KNÜBEL, Georg, D-40229 Düsseldorf (DE); BOMHARD, Andreas, D-40591 Düsseldorf (DE); SCHAPER, Ulf-Armin, D-47804 Krefeld (DE); STALBERG, Theo, D-40789 Monheim (DE); MARKERT, Thomas, D-40591 Düsseldorf (DE)
(86) Internationale Anmeldenummer: EP9403889
(87) Internationale Veröffentlichungsnummer: WO9515320

(56) Entgegenhaltungen:
- WO-A-90/12793
- WO-A-93/02073
- CHEMICAL ABSTRACTS, vol. 105, no. 15, 13. Oktober 1986, Columbus, Ohio, US; abstract no. 134193k, Seite 703 ;

## Beschreibung

### Gebiet der Erfindung

Die Erfindung betrifft ein Verfahren zur Herstellung von 8α,12-Oxido-13,14,15,16-tetranorlabdan sowie die Verwendung spezieller Aluminoschichtsilikate zur Herstellung von 8α,12-Oxido-13,14,15,16-tetranorlabdan.

### Stand der Technik

8α,12-Oxido-13,14,15,16-tetranorlabdan, fortan als Ambroxan bezeichnet, ist ein wertvoller Ambrariechstoff, der in der **Ambra**, einer Stoffwechselausscheidung des Pottwals, enthalten ist (Ullmanns Encyklopädie der technischen Chemie, Band 20, Seite 283, Verlag Chemie Weinheim 1981). Synthetisch kann Ambroxan aus Sclareol durch oxidativen Seitenkettenabbau und nachfolgende Reduktion des gebildeten Lactons (**Sclareolid**) gemäß US 30 50 532 hergestellt werden. Die Überführung von Sclareolid in das geruchlose 8α,12-Dihydroxy-13,14,15,16-tetranorlabdan, fortan kurz als **Diol** bezeichnet, erfolgt in an sich bekannter Weise, beispielsweise durch Reduktion mit Lithiumaluminiumhydrid (Helv. Chim. Acta 1950, 33, 1310), mit Natriumborhydrid (Chem. Abstr. 57, 7316a) oder mit Kaliumborhydrid/Lithiumchlorid-Mischungen (Chem. Abstr. 94, 15913q).

Die cyclisierende Dehydratisierung des Diols zu Ambroxan kann mit sauren Katalysatoren, beispielsweise p-Toluolsulfonsäure, p-Toluolsulfonsäurechlorid, katalytischen Mengen an Schwefelsäure sowie sauren Ionenaustauschern in verschiedenen Lösungsmitteln, beispielsweise Toluol, Hexan, Pyridin, Tetrahydrofuran oder Methanol, vorzugsweise bei Siedetemperatur, durchgeführt werden.

In US 3 029 255 wird die Verwendung von β-Naphthalinsulfonsäure oder Tonerde als Dehydratisierungskatalysatoren bei der Herstellung von Ambroxan beschrieben. Bei diesem Verfahren werden neben Verharzungsprodukten und Olefinen weitere Nebenprodukte erhalten, so daß die Ausbeute an Ambroxan weniger als 77 % beträgt.

JP-A-86/33184 (Takasago) beschreibt ein Verfahren zur Herstellung von Ambroxan, bei der die Cyclisierung der Diolvorstufe durch spezielle Katalysatoren induziert wird. Bei diesen Katalysatoren handelt es sich um säure-beladene aktive Weißerde, Tonerde oder Kieselerde. Als Säuren werden dabei insbesondere Schwefelsäure, Phosphorsäure und Polyphosphorsäure offenbart. Das Takasago-Verfahren hat jedoch Nachteile bezüglich
a) des Umsatzes an Edukt, d. h. an eingesetztem Diol und/oder
b) der Bildung an Dehydratisierungsprodukten (Nebenprodukten) und/oder
c) der Stereoselektivität der Ringschlußreaktion (Ausmaß der iso-Ambrox-Bildung).

Die genannten Nachteile des Takasago-Verfahrens werden durch das in der jüngeren Anmeldung WO 90/12793 beschriebene Verfahren der Anmelderin vermieden. Das Verfahren gemäß WO 90/12793 erfordert jedoch relativ hohe Reaktionstemperaturen und relativ große Mengen an Katalysator. Darüber hinaus läßt sich der spezielle HCl-beladene Katalysator nicht ohne weiteres wiederverwenden.

### Beschreibung der Erfindung

Die Aufgabe der vorliegenden Erfindung bestand in der Entwicklung eines Verfahrens zur Herstellung von Ambroxan durch cyclisierende Dehydratisierung der Diol-Vorstufe, das die Nachteile der aus dem Stand der Technik bekannten Verfahren vermeidet.

Überraschenderweise wurde nun gefunden, daß Ambroxan mit hoher Reinheit und Ausbeute hergestellt werden kann, wenn man die Diol-Vorstufe in einem Lösungsmittel und in Gegenwart von 5 bis 80 Gew.-% - bezogen auf das Diol - Aluminoschichtsilikaten cyclisierend dehydratisiert, die ausgewählt sind aus der Gruppe der K-Katalysatoren und deren Säurebeladung weniger als 100 mval/100g beträgt.

Unter dem Begriff "Säurebeladung" ist im Rahmen der vorliegenden Erfindung derjenige Anteil des Gesamtsäuregehalts der Aluminoschichtsilikate zu verstehen, der nur locker an den Feststoff gebunden ist und sich nach Elution mit Wasser analytisch durch Titration erfassen läßt. In diesem Zusammenhang sei angemerkt, daß die K-Katalysatoren darüber hinaus einen Anteil an Säure enthalten, welcher ionisch an das Gerüst des Katalysators gebunden ist und in wäßriger Dispersion nicht abdissoziiert.

Gegenstand der Erfindung ist ein Verfahren zur Herstellung von 8α,12-0xido-13,14,15,16-tetranorlabdan durch Dehydratisierung von 8α,12-Dihydroxy-13,14,15,16-tetranorlabdan (Diol), wobei man 8α,12-Dihydroxy-13,14,15,16-tetranorlabdan in Gegenwart von 5 bis 80 Gew.-% - bezogen auf das Diol - Aluminoschichtsilikaten cyclisierend dehydratisiert, die ausgewählt sind aus der Gruppe der K-Katalysatoren und deren Säurebeladung weniger als 100 mval/100 g beträgt.

Das erfindungsgemäße Verfahren hat den Vorteil, daß der Katalysator nur in relativ geringen Mengen eingesetzt wird und daß er weitgehend wiederverwendet werden kann. Darüber hinaus erfordert die Durchführung des erfindungsgemäßen Verfahrens nur sehr moderate Temperaturen, so daß durch diese besonders schonende Art der Herstellung die Gefahr der Bildung olfaktorisch unerwünschter Nebenprodukte vermindert ist. Die genannten Vorteile des erfindungsgemäßen Verfahrens gehen nicht zu Lasten der aus der WO 90/12793 bekannten guten Stereoselektivität der Ringschlußreaktion. Dies bedeutet, daß der Anteil des Produkts an den Ambroxan-Isomeren 8-epi- bzw. 9-epi-Ambroxan in derselben Größenordnung liegt, wie bei dem Verfahren gemäß WO 90/12793.

Aluminoschichtsilikate sind Mineralien mit silikatischer Grundstruktur, in denen miteinander über Dipol-Dipol-Wechselwirkungen und Wasserstoffbrückenbindungen verknüpfte Silikatschichten mit teilweise eingelagerten Aluminium³⁺-Ionen vorliegen, und wobei diese zweidimensional unendlichen anionischen Silikatschichten über Kationen einer Zwischenschicht elektrostatisch vernetzt sind. Struktur und Zusammensetzung derartiger Schichtsilikate sind aus dem Stand der Technik bekannt und können der einschlägigen Literatur entnommen werden. Beispiele für Aluminoschichtsilikate sind Talk sowie Tone mit Blattstruktur wie Kaolinit, Montmorillonit, Bentonite und Hectorite.

Der Dehydratisierungskatalysator, d. h. die Aluminoschichtsilikate, wird in dem erfindungsgemäßen Verfahren in einer Menge von - bezogen auf das Diol - 5 bis 80 Gew.-% eingesetzt. Die bevorzugte Menge liegt im Bereich von 15 bis 35 Gew.-%.

Die Ringschlußreaktion wird bei Temperaturen im Bereich von 20 bis 130 °C durchgeführt. Dabei ist der Bereich von 50 bis 110 °C bevorzugt.

In dem erfindungsgemäßen Verfahren setzt man als Dehydratisierungskatalysatoren sogenannte K-Katalysatoren ein. Diese Katalysatoren sind dem Fachmann z. B. aus Nachr. Chem. Tech. Lab. 1985 (33) Nr. 3, S. 202, bekannt. Strukturell handelt es sich bei den K-Katalysatoren um ausgewählte aktivierte Montmorillonit-Katalysatoren, die durch spezielle Säurebehandlung hergestellt wurden. Es handelt sich dabei insbesondere um die Typen KP10, K10, K0, KS, K306, KA0, KA1, KA2 und KA3. K-Katalysatoren, die per se eine höhere Säurebeladung als 100 mval/100g aufweisen, z. B. KSF bzw. KSF/0, bzw. K-Katalysatoren mit herstellungsbedingt geringer Säurebeladung, die jedoch nachträglich mit soviel Säure beladen wurden, daß ihre Säurebeladung oberhalb von 100 mval/100 g liegt, fallen nicht in den Anspruchsbereich der vorliegenden Erfindung.

Die Mikrostruktur der K-Katalysatoren ist in der Literatur nicht explizit beschrieben. Nach den Angaben des Herstellers ist jedoch davon auszugehen, daß je nach Wahl der Herstellparameter (Säurekonzentration, Temperatur, Druck, Reaktionszeit) bei der sauren Aktivierung von Bentonit ein Lösungsvorgang stattfindet, bei dem die Kristallstruktur des Montmorillonits durch Herauslösen von Aluminium-, Eisen- und Magnesiumionen in ganz spezifischer Weise verändert wird (vergleiche Produktionformationsblatt "Was ist Bentonit?" der Firma Südchemie AG).

Die erfindungsgemäß einzusetzenden K-Katalysatoren können gewünschtenfalls zusätzlich mit Säure beladen werden. Die Art der Säure ist dabei an sich keinen speziellen Einschränkungen unterworfen. Besonders bevorzugt sind jedoch Halogenwasserstoffsäuren, insbesondere HCl, sowie Schwefelsäure und Phosphorsäure. Es ist jedoch darauf zu achten, daß die Säurebeladung der eingesetzten K-Katalysatoren unterhalb von 100 mval/100 g, vorzugsweise unterhalb von 80 mval/100 g, liegt.

Das Diol wird üblicherweise in wasserfreier Form eingesetzt. Es ist jedoch auch möglich, ein Diol technischer Qualität mit einem Wassergehalt bis zu ca. 2 Gew.-% einzusetzen.

Ein weiterer Gegenstand der vorliegenden Erfindung ist die Verwendung von Aluminoschichtsilikaten, die ausgewählt sind aus der Gruppe der K-Katalysatoren und deren Säurebeladung weniger als 100 mval/100 g beträgt, zur Herstellung von 8α,12-Oxido-13,14,15,16-tetranorlabdan durch cyclisierende Dehydratisierung von 8α,12-Dihydroxy-13,14,15,16-tetranorlabdan.

Geeignete Lösungsmittel für die cyclisierende Dehydratisierung des Diols sind beispielsweise Toluol und/oder Xylol.

Das während der Dehydratisierung gebildete Wasser kann beispielsweise durch azeotrope Destillation aus dem Reaktionsgemisch entfernt werden. Nach beendeter Dehydratisierung wird das Reaktionsgemisch in an sich bekannter Weise aufgearbeitet.

Das erfindungsgemäße Verfahren kann batchweise oder kontinuierlich durchgeführt werden. Die kontinuierliche Arbeitsweise, z. B. in einem Festbettreaktor, bietet dabei den zusätzlichen Vorteil, daß es keiner speziellen Abtrennung des stückigen Katalysators bedarf.

Die folgenden Beispiele dienen der Erläuterung der Erfindung und sind nicht einschränkend zu verstehen.

### Beispiele

### 1. Allgemeines

### 1.1. Verwendete Substanzen

- **Diol:**: 8α,12-Dihydroxy-13,14,15,16-tetranorlabdan
- **Kat-1:**: Bentonit aktiv, Typ B (Fa. Erbsloh) Säuregehalt: 80 mval H₂SO₄/100 g
- **Kat-2:**: Siliciumdioxid ("Kieselgel 60", Korngröße < 0,063 mm; Fa. Merck), beladen mit Schwefelsäure; Säuregehalt: 200 mval H₂SO₄/100 g
- **Kat-3:**: Aluminiumoxid neutral (Fa. Riedel de Haen), beladen mit Schwefelsäure; Säuregehalt: 200 mval H₂SO₄/100 g
- **Kat-4:**: Aluminiumoxid neutral (Fa. ICN), beladen mit Salzsäure; Säuregehalt: 14 mval HCl/100 g
- **Kat-5:**: Katalysator KP10 (Fa. Süd-Chemie AG); Säuregehalt: ca. 70 mval H₃PO₄/100g
- **Kat-6:**: Katalysator K10 (Fa. Süd-Chemie AG); Säuregehalt: ca. 0 mval H₂SO₄/100 g

### 1.2. Analytik

Zur Quantifizierung der Produkte wurde die gaschramotographische Analyse herangezogen (50 m WG11- Quartz-Kapillare; Injektortemperatur: 220 °C; Detektortemperatur: 250 °C; Ofentemperatur: 80 -> 220 °C bei einer Aufheizgeschwindigkeit von 8 °C/min; Trägergas: Stickstoff; Druck: 20 psi).

### 2. Versuchsbeschreibungen

### 2.1. Herstellung von Ambroxan nach dem Verfahren gemäß JP-A-86/33184

### a) Vergleichsbeispiel V1

51 g n-Heptanol sowie 2,54 g Diol wurden in einem 200-ml-Kolben vorgelegt und das Diol unter Rühren gelöst. Nach dem Zufügen von 0,13 g Katalysator Kat-1 wurde auf einen Druck von 10 mmHg evakuiert und das System bei diesem Druck langsam auf 40 °C erwärmt. Anschließend wurde 3 Stunden bei dieser Temperatur gerührt. Der Katalysator wurde abfiltriert, das Filtrat mit wäßriger Natriumcarbonatlösung gewaschen, das n-Heptanol anschließend im Vakuum abdestilliert.

Die Ausbeute an Ambroxan war mit 7,7 % - bezogen auf eingesetztes Diol - sehr gering; der Anteil an Dehydrierungsprodukten wurde zu 2,5 % - bezogen auf Ambroxan - bestimmt (vergl. Tabelle 1).

### b) Vergleichsbeispiel V2

In einem 2-l-Kolben wurde eine Lösung von 25,4 g Diol in 500 g Xylol zu einer Aufschlämmung von 5 g Katalysator Kat-1 in 200 ml Xylol gegeben. Es wurde auf einen Druck von 50 mmHg evakuiert und das System bei diesem Druck langsam erhitzt. Anschließend wurde 4 Stunden unter Rückfluß gekocht. Der Katalysator wurde anschließend abfiltriert, das Filtrat mit wäßriger Natriumcarbonatlösung gewaschen und schließlich das Xylol abdestilliert.

Die Ausbeute an Ambroxan betrug 32,2 % - bezogen auf eingesetztes Diol; das Produkt enthielt 6,0 % - bezogen auf Ambroxan - an Dehydrierungsprodukten; der Anteil an iso-Ambroxan betrug 1,0 % - bezogen auf eingesetztes Diol (vergl. Tabelle 1).

### Vergleichsbeispiele V3 bis V5

Die für Vergleichsbeispiel V2 beschriebenen Operationen wurden wiederholt, wobei die Reaktionstemperatur sowie Art und Menge des Katalysators variiert wurden. Einzelheiten können Tabelle 2 entnommen werden.

Die bei den Versuchen V3 bis V5 ermittelten Daten können Tabelle 1 entnommen werden.

### 2.2. Herstellung von Ambroxan gemäß der Erfindung

### a) Beispiel B1

25 g technisches Diol (Anteil an reinem Diol : 90 %) wurden in 25 ml Toluol gelöst und mit 8 g des Katalysators KP 10 versetzt. Unter Rühren wurde 4 Stunden auf 70 °C erwärmt. Im Anschluß filtrierte man die Reaktionslösung ab und extrahierte unter Rückfluß den Katalysator mit 300 ml Toluol. Die vereinigten organischen Phasen wurden dann mit 1 %iger Salpetersäure, 1 %iger Natronlauge und abschließend 10 %iger Natriumsulfat-Lösung gewaschen. Im Anschluß wurde im Vakuum bis zur Trocknen eingeengt.

Das Rohprodukt wurde gaschromatographisch analysiert. Die dabei ermittelten Daten können Tabelle 1 entnommen werden.

Der zurückgewonnene Katalysator wurde erneut zur Diol-Cyclisierung eingesetzt. Dabei konnten keinerlei Nachteile in Bezug auf Ausbeute bzw. Nebenproduktbildung festgestellt werden.

### b) Beispiel B2

Beispiel B1 wurde wiederholt, wobei anstelle des Katalysators KP 10 der Katalysator K 10 eingesetzt wurde. Die Ambroxan-Ausbeute betrug 97,7 % (bezogen auf eingesetztes Diol). Weitere Daten können Tabelle 1 entnommen werden.

### 3. Diskussion

Der Vergleich der erfindungsgemäßen Beispiele B1 und B2 mit den Vergleichsbeispielen V1 bis V5 zeigt, daß in bezug auf Ausbeuten und Stereoselektivität deutliche Unterschiede bestehen. Der besseren Übersicht halber sind die o. g. Daten nochmals in Tabelle 1 zusammengestellt; wichtige Versuchsparameter der Beispiele V1 bis V5 und B1 und B2 sind in Tabelle 2 zusammengestellt.

**Tabelle 1**

| Beispiel | Ambroxan^{a)} | iso-Ambroxan^{a)} | | Dehydratisierungsprodukte |
|---|---|---|---|---|
| | | 8-epi | 9-epi | |
| V1 | 7,7 % | n.b.^{c)} | n.b. | 2,5 % |
| V2 | 32,2 % | 0,9 % | 0,1 % | 6,0 % |
| V3 | 68,1 % | 3,0 % | 0,1 % | 17,3 % |
| V4 | 66,3 % | 0,8 % | 0,05 % | 16,1 % |
| V5 | k.U. | n.b. | n.b. | n.b. |
| B1 | 95,6 % | 0,14 | 0,22 | 3,0 % |
| B2 | 97,7 % | 0,15 | 0,26 | 3,0 % |

| | | | | |
|---|---|---|---|---|
| a) Gew.-% bezogen auf eingesetztes Diol b) Gew.-% bezogen auf Ambroxan | | | | |
| c) n. b. = nicht bestimmt d) k. U. = keine Umsetzung | | | | |

**Tabelle 2**

| Beispiel | Temperatur | Katalysator | |
|---|---|---|---|
| | | Art | Menge^{d)} |
| V1 | 40 °C | Kat-1 | 5 % |
| V2 | 130 °C | Kat-1 | 20 % |
| V3 | 130 °C | Kat-2 | 5 % |
| V4 | 130 °C | Kat-3 | 5 % |
| V5 | 50 °C | Kat-4 | 60 % |
| B1 | 70 °C | Kat-5 | 32 % |
| B2 | 70 °C | Kat-6 | 32 % |

| | | | |
|---|---|---|---|
| ^{d)} Gew.-% bezogen auf eingesetztes Diol | | | |

## Patentansprüche

1. Verfahren zur Herstellung von 8α,12-Oxido-13,14,15,16-tetranorlabdan durch Dehydratisierung von 8α,12-Dihydroxy-13,14,15,16-tetranorlabdan (Diol), **dadurch gekennzeichnet**, daß man das Diol in Gegenwart von 5 bis 80 Gew.-% - bezogen auf das Diol - Aluminoschichtsilikaten cyclisierend dehydratisiert, die ausgewählt sind aus der Gruppe der K-Katalysatoren und deren Säurebeladung weniger als 100 mval/100 g beträgt.

2. Verfahren nach Anspruch 1, wobei man den Dehydratisierungskatalysator in einer Menge von - bezogen auf das Diol - 15 bis 35 Gew.-% einsetzt.

3. Verfahren nach Anspruch 1 oder 2, wobei man die Dehydratisierung des Diols bei Temperaturen im Bereich von 50 bis 110 °C durchführt.

4. Verwendung von Aluminoschichtsilikaten, die ausgewählt sind aus der Gruppe der K-Katalysatoren und deren Säurebeladung weniger als 100 mval/100 g beträgt, zur Herstellung von 8α,12-Oxido-13,14,15,16-tetranorlabdan durch cyclisierende Dehydratisierung von 8α,12-Dihydroxy-13,14,15,16-tetranorlabdan.

## Claims

1. A process for the production of 8α,12-oxido-13,14,15,16-tetranorlabdane by dehydration of 8α,12-dihydroxy-13,14,15,16-tetranorlabdane (diol), characterized in that the diol is subjected to cyclizing dehydration in the presence of 5 to 80% by weight, based on the diol, of alumino layer silicates which are selected from the group of K catalysts and which have an acid charge of less than 100 mval/100 g.

2. A process as claimed in claim 1, characterized in that the dehydration catalyst is used in a quantity of - based on the diol - 15 to 35% by weight.

3. A process as claimed in claim 1 or 2, characterized in that the dehydration of the diol is carried out at temperatures of 50 to 110°C.

4. The use of alumino layer silicates which are selected from the group of K catalysts and which have an acid charge of less than 100 mval/100 g for the production of 8α,12-oxido-13,14,15,16-tetranorlabdane by cyclizing dehydration of 8α,12-dihydroxy-13,14,15,16-tetranorlabdane.

## Revendications

1. Procédé de fabrication de 8α,12-oxydo-13,14,15,16-tétranorlabdane par déshydratation de 8α,12-dihydroxy-13,14,15,16-tétranorlabdane (diol),
caractérisé en ce qu'
on déshydrate de façon cyclisante le diol en présence de 5 à 80 % en poids - par rapport au diol - d'aluminosilicates stratifiés, qui sont choisis dans le groupe des catalyseurs K et dont la charge en acide est inférieure à 100 mval/100 g.

2. Procédé selon la revendication 1, dans lequel on utilise le catalyseur de déshydratation en une quantité de 15 à 35 % en poids par rapport au diol.

3. Procédé selon la revendication 1 ou 2, dans lequel on effectue la déshydratation du diol à des températures comprises entre 50 et 110°C.

4. Utilisation d'aluminosilicates stratifiés qui sont choisis dans le groupe des catalyseurs K et dont la charge en acide est inférieure à 100 mval/100 g, pour la fabrication de 8α,12-oxydo-13,14,15,16-tétranorlabdane par déshydratation cyclisante de 8α,12-dihydroxy-13,14,15,16-tétranorlabdane.
